# EUROPEAN PATENT APPLICATION

(11) **EP 3 460 070 A1**
(43) Date of publication of application: **27.03.2019**
(21) Application number: 17001590.3
(22) Date of filing: 22.09.2017
(51) Int. Cl.: C12Q 1/56, G01N 33/52

(54) **IMPROVED DETECTION OF ANTICOAGULANTS IN BODY FLUIDS**

(71) Applicant: DOASENSE GmbH, 69123 Heidelberg (DE)
(72) Inventor: Job, Harenberg, Heidelberg 69126 (DE)
(74) Representative: Müller-Boré & Partner Patentanwälte PartG mbB

(57) **Abstract**

The present invention relates to the use of excitation light having a wavelength in the range of 340 nm to 400 nm for detecting at least one anticoagulant in a sample, wherein the sample is derived from a body fluid and does not contain citrated blood plasma, and to a method related thereto.

## Description

The present invention relates to the use of excitation light having a wavelength in the range of 340 nm to 400 nm for detecting at least one anticoagulant in a sample, wherein the sample is derived from a body fluid and does not contain citrated blood plasma, and to a method related thereto.

Coagulation tests of blood are conducted on plasma samples from humans/animals by making blood samples incoagulable with sodium citrate (volume ratio blood/anticoagulant 9:1). To conduct the determination of individual measurement values of the blood coagulation, blood is centrifuged and the cells (in the sediment) are separated from the liquid blood components (plasma). To measure the coagulation time of plasma, calcium chloride and an activator of the coagulation system are added to the plasma. In special methods for determining individual blood clotting factors or exogenous inhibitors, photometric proof is used. In these methods, the colorant para-nitroaniline is released by an exogenously added coagulation enzyme (e.g. factor Xa, thrombin) from a chromogenic substrate such as N-benzoyl-L-isoleucyl-L-glutamyl-L-glycyl-L-arginine-para-nitroaniline, N-(para-tosyl)-glycyl-L-prolyl-L-arginine-para-nitroanilide acetate and others, and the activity/concentration of anticoagulants (e.g. factor Xa inhibitor, thrombin inhibitor) is measured in a concentration-dependent manner. The result of the enzymatic reaction is a linear or sigmoidal decrease of the release of the colorant para-nitroaniline, which depends on the concentration of the anticoagulant. Typically, the amount of released para-nitroaniline is determined by means of photometry.

Up to now, the photometric investigation of the activity/concentration of anticoagulants in samples from humans/animals has the drawback of exhibiting strong signal fluctuations specifically at low concentrations, which makes it difficult to reliably evaluate as to whether anticoagulants such as factor Xa inhibitors or thrombin inhibitors are present in the sample under investigation. For the routine use of photometric applications in a clinical laboratory, it is, however, required to achieve both a high selectivity and a high specificity in the detection of anticoagulants.

In view of the above, the technical problem underlying the present invention is to provide new means for an efficient detection of anticoagulants, which overcome the shortcomings of the protocols known in the art.

The solution to the above technical problem is achieved by providing the embodiments characterized in the claims.

In particular, in a first aspect, the present invention relates to the use of excitation light having a wavelength in the range of 340 nm to 400 nm for detecting at least one anticoagulant in a sample, wherein the sample is derived from a body fluid and does not contain citrated blood plasma.

When using excitation light having a wavelength in the above-indicated range, it is possible to detect, by means of photometry, anticoagulants such as factor Xa inhibitors or thrombin inhibitors in a sample both with a high selectivity and with a high specificity.

According to the present invention, the light source used for providing the excitation light is not particularly limited as long as it is capable of emitting light, which has a wavelength in the range of 340 nm to 400 nm. Herein, suitable light sources include, but are not limited to light-emitting diodes (LEDs), which have the emission maximum between 340 nm and 400 nm. Such LEDs are commercially available, e.g. with an emission maximum of around 365 nm or around 385 nm. However, it is also within the scope of the present invention to use a light source which has an emission maximum outside the range of 340 nm to 400 nm, provided that the emission intensity in the above-indicated range is sufficient for detecting at least one anticoagulant in a sample, as explained further below. In such a case, as required, suitable band-pass filters may be employed so as to narrow the emission range of the light source.

In a preferred embodiment of the present invention, excitation light having a wavelength in the range of 360 nm to 395 nm is used. More preferably, excitation light having a wavelength in the range of 370 nm to 390 nm is used.

Herein, the term "excitation light" is not to be construed as a limitation with respect to the term "light". The term "excitation" shall merely indicate the purpose thereof. As required, the term "light" may be used instead of the term "excitation light".

According to the present invention, the term "anticoagulant", also commonly referred to as blood thinner, means a chemical substance, which prevents or reduces the coagulation of blood, thereby prolonging the clotting time. Numerous anticoagulants are known in the art. Beside the traditional ones (warfarin, other coumarins and heparins) still being widespreadly used, new agents have been introduced since the early 2000s, which are collectively referred to as novel oral anticoagulants (NOACs) or directly acting oral anticoagulants (DOACs). These agents *inter alia* include a direct and immediate inhibition of factor Xa and thrombin, and possess numerous improved pharmacologic advantages over vitamin-K antagonists. They have been shown to be almost as effective and safe or even more effective and safer for treatment of patients suffering from atrial fibrillation and venous thromboembolism as compared to the traditional anticoagulants.

Thus, in a preferred embodiment of the present invention, the at least one anticoagulant is a direct factor Xa inhibitor or a direct thrombin inhibitor. The direct factor Xa inhibitor can form a complex with the corresponding coagulation enzyme factor Xa, and the direct thrombin inhibitor can form a complex with the corresponding coagulation enzyme thrombin. Herein, the terms "coagulation enzyme" and "blood clotting factor" are used synonymously.

According to the present invention, the terms "factor Xa" and "thrombin" do not underlie a specific restriction and may include any activated factor Xa or thrombin obtained from a natural source or via recombinant DNA technology, or a biologically active derivative thereof. As used herein, the term "biologically active derivative" includes any derivative of a protein, protein complex or polypeptide having substantially the same functional and/or biological properties of factor Xa or thrombin, such as binding properties, and/or the same structural basis, such as a peptidic backbone. The polypeptide sequences of the functionally active derivatives may contain deletions, additions and/or substitutions of amino acids whose absence, presence and/or substitution, respectively, do not have any substantial negative impact on the activity of the polypeptide, e.g. amino acids which are located in a part of the polypeptide sequence that does not contribute to the biological activity of the protein. Minor deletions, additions and/or substitutions of amino acids of the respective polypeptide sequences, which are not altering the biological activity of said polypeptide are also included in the present invention as biologically active derivatives.

A factor Xa or thrombin obtained from a natural source may be any factor Xa or thrombin isolated from a blood product derived from a mammal. In a preferred embodiment of the present invention, the mammal is selected from the group consisting of human, mouse, rat, pig, cat, dog, horse, goat, cattle, cow, and monkey and/or others. In a particularly preferred embodiment, the factor Xa or thrombin is isolated from a blood product of a human. In a preferred embodiment of the present invention, the factor Xa or thrombin is isolated from a blood product selected from the group consisting of whole blood, serum, or plasma, including isolated blood compounds and processed blood products. A factor Xa obtained from a natural source may be a factor Xa obtained by isolating factor X from a blood product as defined above and subsequently activating the isolated factor X to become factor Xa, e.g. by using any thromboplastin or by using viper venom, such as Russell's viper venom. Similarly, a thrombin obtained from a natural source may be a thrombin obtained by isolating from a blood product as defined above and subsequently activating the isolated thrombin, e.g. by using any thromboplastin or by using viper venom, such as Russell's viper venom.

The factor Xa or thrombin according to the present invention may be produced by any method known in the art. This may include any method known in the art for the production of recombinant DNA by genetic engineering, e.g. via reverse transcription of RNA and/or amplification of DNA. This includes methods which comprise the recombinant production of factor X and the subsequent activation of factor X, e.g. by using thromboplastin or by using Russell's viper venom, in order to obtain factor Xa. In the same way, this includes methods which comprise the recombinant production of thrombin and the subsequent activation of prothrombin, e.g. by using thromboplastin or by using Russell's viper venom, in order to obtain thrombin.

For example, the recombinant DNA coding for factor X, e.g. a plasmid, or the recombinant DNA coding for prothrombin, e.g. a plasmid, may also contain a DNA sequence encoding a selectable marker for selecting the cells, which have been successfully transfected with the plasmid. In an example of the present invention, the plasmid may also confer resistance to a selectable marker, e.g. to the antibiotic drug G418, by delivering a resistance gene, e.g. the neo resistance gene conferring resistance to G418.

The production of factor Xa or thrombin may include any method known in the art for the introduction of recombinant DNA into eukaryotic cells by transfection, e.g. via electroporation or microinjection. For example, the recombinant expression of human factor X or thrombin can be achieved by introducing an expression plasmid containing the human factor X or prothrombin encoding DNA sequence under the control of one or more regulating sequences, such as a strong promoter, into a suitable host cell line by an appropriate transfection method resulting in cells having the introduced sequences stably integrated into the genome. The calcium-phosphate coprecipitation method is an example of a transfection method, which may be used according to the present invention.

The production of factor Xa or thrombin may also include any method known in the art for the cultivation of said transformed cells, e.g. in a continuous or batchwise manner, and the expression of the factor X or thrombin, e.g. constitutive or upon induction. In one specific example of the present invention, the nucleic acid coding for factor X or thrombin contained in the host organism is expressed via an expression mode selected from the group consisting of induced, transient, and permanent expression. Any expression system known in the art or commercially available can be employed for the expression of a recombinant nucleic acid encoding factor X or thrombin, including the use of regulatory systems such as suitable, e.g. controllable, promoters, enhancers etc.

The production of factor Xa or thrombin may also include any method known in the art for the isolation of the protein, e.g. from the culture medium or by harvesting the transformed cells. For example, the factor X-producing cells or the thrombin-producing cells can be identified by isolating single-cell derived populations, i.e. cell clones, via dilution after transfection and optionally via addition of a selective drug to the medium. After isolation, the identified cell clones may be cultivated until confluency in order to enable the measurement of the factor X or thrombin content of the cell culture supernatant by enzyme-linked immuno-sorbent assay (ELISA) technique.

Additionally, the production of factor Xa or thrombin may include any method known in the art for the purification of factor X/Xa or thrombin, e.g. via anion exchange chromatography or affinity chromatography. In a preferred embodiment, factor X or thrombin can be purified from cell culture supernatants by semi-affinity calcium-dependent anion exchange chromatography, e.g. in an endotoxin-free system. The purified factor X/Xa or thrombin may be analyzed by methods known in the art for analyzing recombinant proteins, e.g. the ELISA technique. In addition, the protein integrity and activity may be assessed. It is within the knowledge of a person skilled in the art to select the optimal parameters, such as the buffer system, the temperature and the pH for the respective detection system to be used.

In one specific example of the present invention, factor X or thrombin is expressed in a host cell type with the ability to perform posttranslational modifications. The ability to perform posttranslational modifications of factor X or thrombin expressing host cell lines may be, for example, analyzed by mass-spectrometric analysis.

The host cell type used for the recombinant production of factor Xa or thrombin may be any mammalian cell, preferably with the ability to perform posttranslational modifications of factor X or thrombin. There is no particular limitation to the media, the reagents and the conditions used for culturing the cells in the cell culture used for the recombinant production of factor Xa or thrombin, including culturing the cells in a continuous or batchwise manner. The desired factor X or thrombin protein which has been expressed by the cells, and which, dependent on the transfection/vector-system used, is contained in the cells or secreted into the medium for culturing cells, can be isolated/recovered from the cell culture using methods known in the art, as mentioned herein before.

In particular, the term "factor Xa" as used herein comprises any factor Xa which is obtained by producing and isolating factor X according to any method available in the prior art and disclosed herein followed by a subsequent activation of factor X, e.g. by using thromboplastin or Russell's viper venom, and the term "thrombin" as used herein comprises any thrombin which is obtained by producing and isolating thrombin according to any method available in the prior art and disclosed herein followed by a subsequent activation of prothrombin, e.g. by using thromboplastin or Russell's viper venom.

In a preferred embodiment of the present invention, the at least one anticoagulant is a direct factor Xa inhibitor or a direct thrombin inhibitor. As used herein, the terms "direct factor Xa inhibitor" and "direct thrombin inhibitor" relate to any naturally occurring or artificially synthesized inhibitor of factor Xa activity and thrombin activity, respectively. Herein, the direct factor Xa inhibitor may be selected from the group consisting of rivaroxaban, apixaban, edoxaban, betrixaban and otamixaban, and the direct thrombin inhibitor may be selected from the group consisting of dabigatran, ximelagatran, argatroban, hirudins and modified hirudins, without being limited to the aforementioned agents. In a preferred embodiment of the present invention, the direct factor Xa inhibitor is selected from the group consisting of apixaban, edoxaban and rivaroxaban. In another preferred embodiment of the present invention, the direct thrombin inhibitor is dabigatran.

According to the present invention, the number of different anticoagulants contained in the sample, such as direct factor Xa inhibitors or direct thrombin inhibitors, is not specifically limited. In particular, the sample may contain both direct factor Xa inhibitor(s) and direct thrombin inhibitor(s).

As mentioned above, the present invention is directed to the detection of at least one anticoagulant in a sample, wherein the sample is derived from a body fluid, i.e. contains a body fluid, and does not contain citrated blood plasma. Preferably, the body fluid is selected from the group consisting of serum and urine, and more preferably, the body fluid is urine. The body fluid may be taken from a mammal, preferably from a mammal selected from the group consisting of human, mouse, rat, pig, cat, dog, horse, goat, cattle, cow, and monkey and/or others. In a preferred embodiment of the present invention, the sample is taken from a human. Methods for obtaining the above samples are known in the art.

In a further aspect, the present invention relates to a method for detecting at least one anticoagulant in a sample, wherein the method comprises the steps of:
(a) providing a sample containing at least one anticoagulant, wherein the sample is derived from a body fluid and does not contain citrated blood plasma;
(b) providing a composition containing at least one blood clotting factor;
(c) providing a composition containing a chromogenic substrate conjugated to a detectable substance;
(d) mixing the sample of step (a) with the composition of step (b) and the composition of step (c) under conditions which allow the binding of the at least one anticoagulant to the at least one blood clotting factor, and which allow the at least one blood clotting factor to release the detectable substance from the chromogenic substrate; and
(e) measuring the amount of released detectable substance photometrically using excitation light having a wavelength in the range of 340 nm to 400 nm.

All definitions and limitations provided above for the use according to the present invention equally apply to the method according to the present invention. In the same way, all definitions and limitations provided below for the method according to the present invention equally apply to the use according to the present invention.

In particular, this means that in the above-defined method, the at least one anticoagulant preferably is a direct factor Xa inhibitor or a direct thrombin inhibitor. Specifically, the direct factor Xa inhibitor may be selected from the group consisting of rivaroxaban, apixaban, edoxaban, betrixaban and otamixaban, and the direct thrombin inhibitor may be selected from the group consisting of dabigatran, ximelagatran, argatroban, hirudins and modified hirudins, without being limited to the aforementioned agents. As the direct factor Xa inhibitor, apixaban, edoxaban and rivaroxaban are preferred, and as the direct thrombin inhibitor, dabigatran is preferred. In addition, the body fluid is preferably selected from the group consisting of serum and urine, and more preferably, is urine.

In the following, steps (a) to (e) of the method for detecting at least one anticoagulant in a sample according to the present invention are described in more detail.

In step (a) of the method as defined above, a sample containing at least one anticoagulant is provided.

According to the present invention, the sample is derived from a body fluid, i.e. contains a body fluid, and does not contain citrated blood plasma. For example, the sample is taken from a patient to which the at least one anticoagulant has been administered before step (a) of the method for detecting at least one anticoagulant in a sample according to the present invention. The patient can be selected from the group consisting of human, mouse, rat, pig, cat, dog, horse, goat, cattle, cow, and monkey and/or others. Preferably, the patient is a human being.

In a preferred embodiment of the above-defined method, the sample is pre-purified before step (a). In a more preferred embodiment, the pre-purification comprises the step of removing impurities that prevent the anticoagulant, such as the direct factor Xa inhibitor or the direct thrombin inhibitor, from binding to the corresponding coagulation enzyme, such as factor Xa or thrombin.

In step (b) of the method as defined above, a composition containing at least one blood clotting factor is provided.

Again, the terms "blood clotting factor" and "coagulation enzyme" are used herein in a synonymous manner.

In this context, it is clear to the skilled person that the blood clotting factor provided with the composition in step (b) has to match the anticoagulant provided with the sample in step (a) of the above-defined method. In particular, in case the at least one anticoagulant is a direct factor Xa inhibitor, the at least one blood clotting factor is factor Xa. Similarly, in case the at least one anticoagulant is a direct thrombin inhibitor, the at least one blood clotting factor is thrombin.

The composition provided in step (b) of the method according to the present invention may contain suitable buffer salts along with the at least one blood clotting factor, as required. In a preferred embodiment of the present invention, the composition containing the at least one blood clotting factor is isotonic within the physiological limits of the pH and may be of normal or low ionic strength.

In step (c) of the method as defined above, a composition containing a chromogenic substrate conjugated to a detectable substance is provided.

According to the present invention, the term "chromogenic substrate conjugated to a detectable substance" means that the chromogenic substrate is covalently linked to at least one detectable substance. The term "detectable substance" does not include any particular limitation as long as the detectable substance is capable of being excited with the above-specified excitation light, such as a colorant absorbing the above-specified excitation light. In a preferred embodiment of the present invention, the detectable substance is para-nitroaniline.

The chromogenic substrate conjugated to a detectable substance provided in step (c) of the above-defined method is any chromogenic substrate conjugated to a detectable substance, which can be cleaved by the at least one blood clotting factor, such as factor Xa or thrombin, so that the detectable substance, e.g. para-nitroaniline, is released from the chromogenic substrate.

For example, the conjugation of the chromogenic substrate to the detectable substance is via the linker isoleucine-glutamine-glycine-arginine-X (Ile-Glu-Gly-Arg-X) or via the linker isoleucine-aspartic acid-glycine-arginine-X (Ile-Asp-Gly-Arg-X), wherein the residue X is any amino acid except of proline. Besides, the chromogenic substrate conjugated to a detectable substance may be an amino acid sequence which contains the sequence Ile-Glu-Gly-Arg-X or Ile-Asp-Gly-Arg-X, wherein the residue X is any amino acid except of proline, at the site where the detectable substance binds, provided that the structure of the chromogenic substrate conjugated to a detectable substance is such that the at least one blood clotting factor cleaves the sequence Ile-Glu-Gly-Arg-X or Ile-Asp-Gly-Arg-X under physiological conditions at room temperature.

Examples of chromogenic substrates conjugated to a detectable substance include, without limitation, Ile-Glu-Gly-Arg para-nitroanilide hydrochloride, N-benzoyl-Ile-Glu-Gly-Arg para-nitroanilide acetate, N-benzoyl-L-isoleucyl-L-glutamyl-L-glycyl-L-arginine-para-nitroaniline, N-benzoyl-Ile-Glu-Gly-Arg para-nitroanilide hydrochloride, N-benzoyl-Ile-Glu-Gly-Arg para-nitroanilide, Boc-Ile-Glu-Gly-Arg-7-amido-4-methylcoumarin hydrochloride, 4-nitrophenyl 4-guanidinobenzoate hydrochloride, benzyl-isoleucine-glutamine-glycine-arginine-para-nitroaniline hydrochloride, N-(para-tosyl)-glycyl-L-prolyl-L-arginine-para-nitroanilide acetate, and bis-para-tosyl-L-glycyl-L-prolyl-L-arginine amide.

In case the at least one anticoagulant is a direct factor Xa inhibitor, the chromogenic substrate conjugated to a detectable substance is preferably selected from the group consisting of Ile-Glu-Gly-Arg para-nitroanilide hydrochloride, N-benzoyl-Ile-Glu-Gly-Arg para-nitroanilide acetate, N-benzoyl-L-isoleucyl-L-glutamyl-L-glycyl-L-arginine-para-nitroaniline, N-benzoyl-Ile-Glu-Gly-Arg para-nitroanilide hydrochloride, N-benzoyl-Ile-Glu-Gly-Arg para-nitroanilide, Boc-Ile-Glu-Gly-Arg-7-amido-4-methylcoumarin hydrochloride, and 4-nitrophenyl 4-guanidinobenzoate hydrochloride. More preferably, in case of the direct factor Xa inhibitor, the chromogenic substrate conjugated to a detectable substance is N-benzoyl-L-isoleucyl-L-glutamyl-L-glycyl-L-arginine-para-nitroaniline or a derivative thereof.

In case the at least one anticoagulant is a direct thrombin inhibitor, the chromogenic substrate conjugated to a detectable substance is preferably selected from the group consisting of benzyl-isoleucine-glutamine-glycine-arginine-para-nitroaniline hydrochloride, N-(para-tosyl)-glycyl-L-prolyl-L-arginine-para-nitroanilide acetate, and bis-para-tosyl-L-glycyl-L-prolyl-L-arginine amide. More preferably, in case of the direct thrombin inhibitor, the chromogenic substrate conjugated to a detectable substance is N-(para-tosyl)-glycyl-L-prolyl-L-arginine-para-nitroanilide acetate or a derivative thereof.

In this context, the term "derivative" means that the chromogenic substrate conjugated to a detectable substance may be chemically modified as long as the respective sequence can be cleaved by the at least one blood clotting factor, e.g. factor Xa or thrombin.

In step (d) of the method as defined above, the sample of step (a) is mixed with the composition of step (b) and the composition of step (c) under conditions which allow the binding of the at least one anticoagulant to the at least one blood clotting factor, and which allow the at least one blood clotting factor to release the detectable substance from the chromogenic substrate.

Herein, a buffer solution may be used in addition, which allows the binding of the at least one anticoagulant to the at least one blood clotting factor so as to form an anticoagulant-blood clotting factor-complex, and which allows the at least one blood clotting factor to release the detectable substance from the chromogenic substrate. If a buffer solution is used in step (d) of the above-defined method, the buffer solution may contain any compound which does not negatively affect the formation of the anticoagulant-blood clotting factor-complex, and which does not negatively affect the release of the detectable substance from the chromogenic substrate by the non-complexed blood clotting factor. In a preferred embodiment of the above-defined method, the conditions in step (d) comprise the use of a buffer solution which is isotonic and within the physiological limits of the pH. It may be of normal or low ionic strength. The buffer salt which may be used in step (d) of the method according to the present invention is not particularly limited as long as it does not adversely affect the formation of the complex and the release of the detectable substance.

Step (d) of the method for detecting at least one anticoagulant in a sample according to the present invention may be carried out under any conditions suitable for binding of the at least one anticoagulant to the at least one blood clotting factor, and which allow the at least one blood clotting factor to release the detectable substance from the chromogenic substrate without any limitation. This comprises e.g. any suitable temperature, time period and agitation of the buffer solution. In a preferred embodiment of the present invention, the incubation is carried out at a temperature ranging from about 20°C to about 30°C for about 10 minutes. In a more preferred embodiment of the present invention, the incubation is carried out at 22°C for about 10 minutes.

In step (e) of the method as defined above, the amount of released detectable substance is photometrically measured using excitation light having a wavelength in the range of 340 nm to 400 nm. For photometrically measuring the amount of released detectable substance, it is preferable to use a reflectance photometer.

The specific conditions for measuring the amount of released detectable substance in step (e) of the method according to the present invention depend on the system under investigation, such as the specific anticoagulant with its corresponding blood clotting factor, as well as the specific chromogenic substrate conjugated to a detectable substance. It is within the knowledge of the person skilled in the art to select the optimal parameters, such as the buffer system, the temperature and the pH for the respective system to be investigated.

As mentioned above, the amount of released detectable substance is measured photometrically using excitation light having a wavelength in the range of 340 nm to 400 nm, preferably in the range of 360 nm to 395 nm, and more preferably in the range of 370 nm to 390 nm. In this context, all definitions and limitations provided above for the use according to the present invention, including the specifications relating to the excitation light, apply to the method according to the present invention in an equal manner.

In a preferred embodiment of the present invention, the detectable substance of the chromogenic substrate conjugated to a detectable substance is para-nitroaniline. The amount of released para-nitroaniline can then be effectively monitored by means of photometry using excitation light having a wavelength in the range of 340 to 400 nm, preferably in the range of 360 nm to 395 nm, and more preferably in the range of 370 nm to 390 nm.

In particular, when measuring the amount of released detectable substance with a photometer, preferably with a reflectance photometer, while using excitation light having a wavelength in the above-indicated range, both high sensitivity and high specificity can be achieved in the detection of the at least one anticoagulant in the sample under investigation.

In a preferred embodiment of the present invention, the method for detecting at least one anticoagulant in a sample is a point-of-care testing. Preferably, in this embodiment, the composition provided in step (b), i.e. the composition containing at least one blood clotting factor, is immobilized on a test strip, and the composition provided in step (c), i.e. the composition containing a chromogenic substrate conjugated to a detectable substance, is also immobilized on the test strip, but on a different site thereof. The mixing of the sample of step (a) with the composition of step (b) and the composition of step (c) in step (d) of the above-defined method is accomplished by applying the sample to be investigated on the test strip, on which the composition containing the at least one blood clotting factor and the composition containing the chromogenic substrate conjugated to a detectable substance are immobilized, i.e. by dipping the test strip into the sample to be investigated, e.g. for about 2 to 3 seconds. The photometric measurement of the amount of released detectable substance in step (e) of the above-defined method is accomplished by inserting the test strip into an instrument being suitable for photometrically measuring the amount of released detectable substance, also referred to as a reader, which is a photometer, preferably a reflectance photometer, and measuring the amount of released detectable substance using excitation light having a wavelength in the above-indicated range. In a preferred embodiment, the instrument is a transportable, portable or handheld instrument.

Although both the composition of step (b) and the composition of step (c) are immobilized on the test strip, the at least one blood clotting factor does not readily release the detectable substance from the chromogenic substrate. Due to the immobilization on different sites of the test strip, such release cannot take place until the (liquid) sample of step (a) containing the at least one anticoagulant is applied on the test strip, on which the at least one blood clotting factor and the chromogenic substrate conjugated to a detectable substance are immobilized.

In a specific embodiment of the present invention, the method for detecting at least one anticoagulant in a sample is a method for point-of-care testing as defined herein, wherein the sample is urine, the at least one anticoagulant is either a direct factor Xa inhibitor, e.g. rivaroxaban, with N-benzoyl-L-isoleucyl-L-glutamyl-L-glycyl-L-arginine-para-nitroaniline as the chromogenic substrate conjugated to a detectable substance, or a direct thrombin inhibitor, e.g. dabigatran, with N-(para-tosyl)-glycyl-L-prolyl-L-arginine-para-nitroanilide acetate as the chromogenic substrate conjugated to a detectable substance. Furthermore, in the above specific embodiment, the composition provided in step (b) and the composition provided in step (c) are immobilized on a test strip.

Preferably, the amount of released para-nitroaniline is then photometrically measured using excitation light having a wavelength in the range of 340 nm to 400 nm. As the composition provided in step (b) and the composition provided in step (c) are immobilized on the test strip, the amount of released para-nitroaniline can be measured directly after insertion of the test strip into the test instrument using a reflectance photometer.

In a preferred embodiment of the present invention, the above-defined method further comprises a step of quantifying the amount of the at least one anticoagulant in the sample under investigation after having photometrically measured, preferably with a reflectance photometer, the amount of released detectable substance. The amount of the at least one anticoagulant in the sample correlates with the amount of released detectable substance. That is, the amount of released detectable substance decreases with an increase of the at least one anticoagulant in the sample. Herein, the quantification of the amount of the at least one anticoagulant in the sample can be accomplished by standard methods known in the art. In a preferred embodiment of the present invention, the amount of the at least one anticoagulant in the sample to be quantified is calculated from a calibration curve obtained for the at least one anticoagulant in a defined amount using a photometer, preferably a reflectance photometer.

As already mentioned, in a preferred embodiment of the present invention, the amount of released detectable substance is measured in step (e) of the above-defined method using reflectance photometry. This approach is particularly suitable when using a test strip, on which the composition provided in step (b) and the composition provided in step (c) are immobilized. In contrast to conventional transmission photometry, wherein the excitation light transmits the (transparent) sample and is then detected, it is here the excitation light reflected from the (opaque) sample, which is detected. In this regard, the more anticoagulant is present in the sample, the less detectable substance is released from the chromogenic substrate, which may absorb the excitation light. In turn, this means that the intensity of the reflected excitation light increases when the amount of the at least one anticoagulant in the sample increases. Advantageously, when applying reflectance photometry in connection with the test strip, it is not required to use a cuvette or another container filled with the (liquid) sample, thereby rendering the measurement less time-consuming.

Optionally, in order to suppress fluorescence light which might originate from the released detectable substance upon absorption of excitation light, at least one optical filter may be provided in step (e) of the above-defined method. For this purpose, any suitable band-pass and/or short-pass filter may be employed depending on the optical properties of the detectable substance. The only prerequisite in this respect is that the at least one optical filter allows for a transmission of the excitation light while blocking the longwave fluorescence light. Typical filters which may be used herein include UV band-pass filters, e.g. made of UG11 glass, as available from Schott AG.

The present invention allows to detect anticoagulants, such as direct factor Xa inhibitors or direct thrombin inhibitors, in samples, such as urine, both with a high selectivity and with a high specificity. The reason therefor lies in the use of excitation light having a wavelength in the range of 340 nm to 400 nm for detecting the at least one anticoagulant in the sample under investigation.

The Figures show:

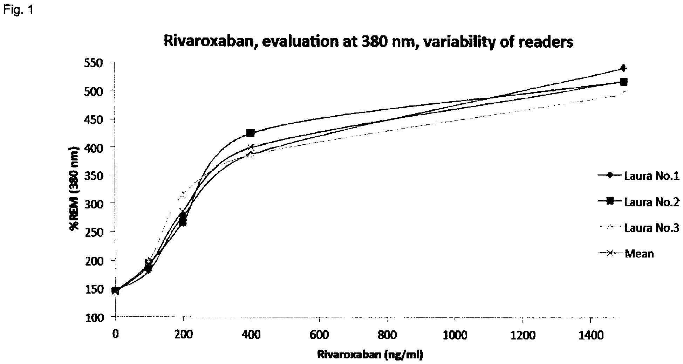

Fig. 1 shows the photometrically measured intensity (in reflectance units, %REM) obtained from a sample (native urine) containing the direct factor Xa inhibitor rivaroxaban as a function of its concentration (in ng/mL), measured with excitation light having a wavelength of 380 nm using reflectance photometry. Three measurement series taken with different readers ("Laura No. 1", "Laura No. 2", and "Laura No. 3") are shown, including the mean calculated therefrom.

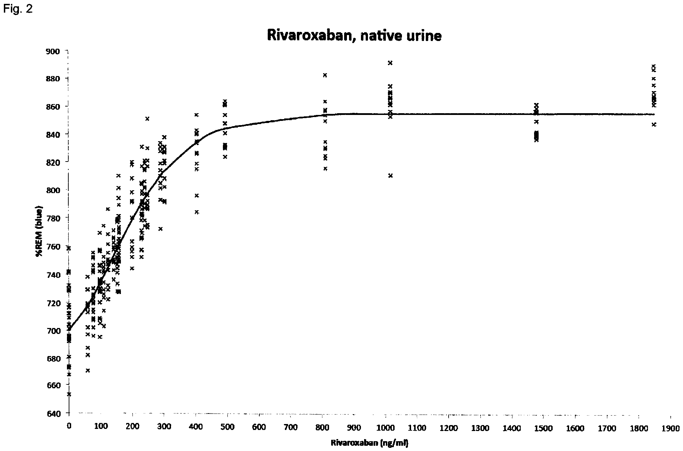

Fig. 2 shows the photometrically measured intensity (in reflectance units, %REM) obtained from a sample (native urine) containing the direct factor Xa inhibitor rivaroxaban as a function of its concentration (in ng/mL), measured with excitation light having a wavelength of 470 nm using reflectance photometry. A strong fluctuation of the measured intensity can be observed.

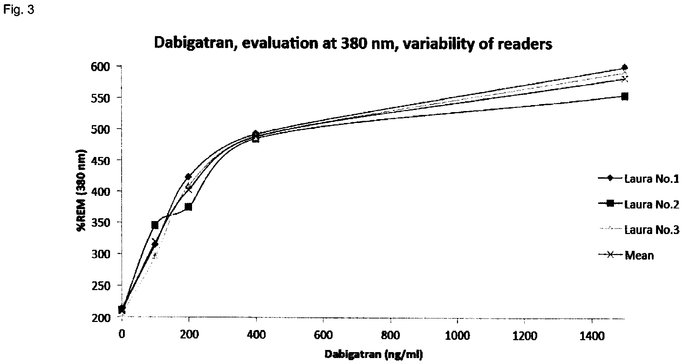

Fig. 3 shows the photometrically measured intensity (in reflectance units, %REM) obtained from a sample (native urine) containing the direct thrombin inhibitor dabigatran as a function of its concentration (in ng/mL), measured with excitation light having a wavelength of 380 nm using reflectance photometry. Three measurement series taken with different readers ("Laura No. 1", "Laura No. 2", and "Laura No. 3") are shown, including the mean calculated therefrom.

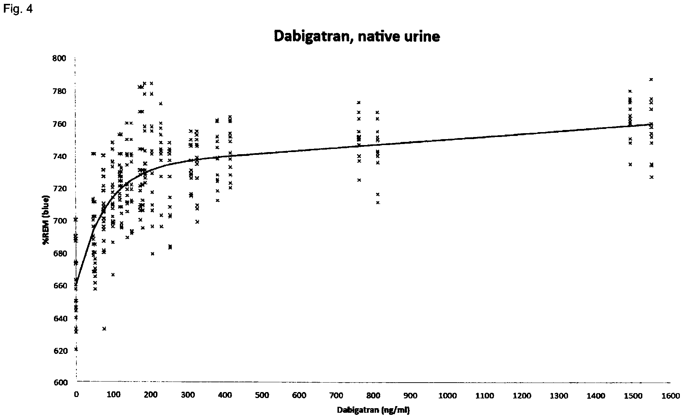

Fig. 4 shows the photometrically measured intensity (in reflectance units, %REM) obtained from a sample (native urine) containing the direct thrombin inhibitor dabigatran as a function of its concentration (in ng/mL), measured with excitation light having a wavelength of 470 nm using reflectance photometry. A strong fluctuation of the measured intensity can be observed.

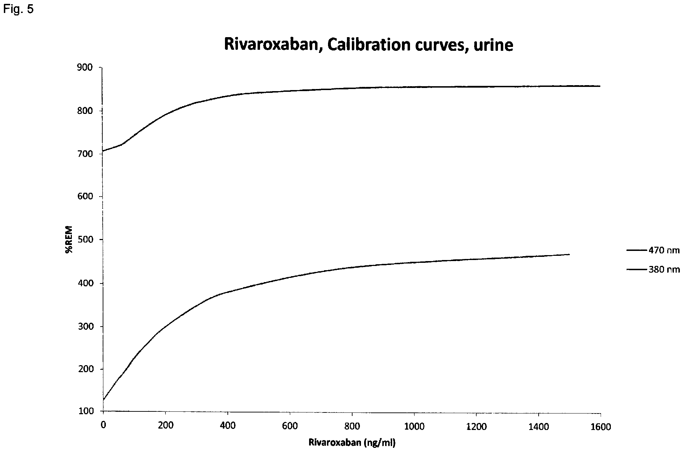

Fig. 5 shows calibration curves for the direct factor Xa inhibitor rivaroxaban as a function of its concentration (in ng/mL), measured with excitation light having a wavelength of either 380 nm or 470 nm using reflectance photometry. When using excitation light having a wavelength of 380 nm, the photometrically measured intensity extends over a larger detection range (in reflectance units, %REM).

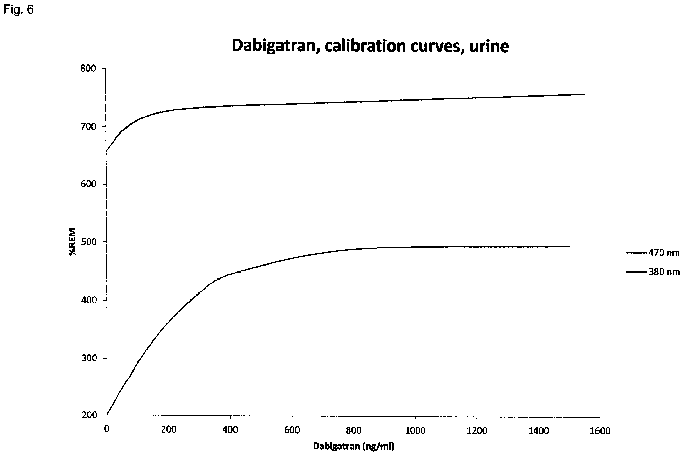

Fig. 6 shows calibration curves for the direct thrombin inhibitor dabigatran as a function of its concentration (in ng/mL), measured with excitation light having a wavelength of either 380 nm or 470 nm using reflectance photometry. When using excitation light having a wavelength of 380 nm, the photometrically measured intensity extends over a larger detection range (in reflectance units, %REM).

### Examples

The present invention will be further illustrated by the way of Examples. But the present invention is not to be construed as being limited to the Examples provided below.

Using a modified reflectance photometer, hereinafter also referred to as reader (Laura Smart (LS), ERBA Lachema), native urine samples were investigated using excitation light having either a wavelength of 380 nm or a wavelength of 470 nm. Herein, the samples under investigation contained the direct factor Xa inhibitor rivaroxaban or the direct thrombin inhibitor dabigatran. For each anticoagulant, samples with different concentrations were provided.

Test strips were used on which both the blood clotting factor (factor Xa or thrombin) and the respective chromogenic substrate conjugated to a detectable substance were immobilized, i.e. N-benzoyl-L-isoleucyl-L-glutamyl-L-glycyl-L-arginine-para-nitroaniline together with factor Xa, and N-(para-tosyl)-glycyl-L-prolyl-L-arginine-para-nitroanilide acetate together with thrombin. After dipping the test strip into the urine sample to be investigated for 2 to 3 seconds, incubation was allowed for 10 min. Then, the test strip was inserted into the reader. In total, three different readers ("Laura No. 1", "Laura No. 2", and "Laura No. 3") were used in order to check for any device-specific issues.

In order to suppress fluorescence light which might originate from the released detectable substance (para-nitroaniline), a UV band-pass filter was used made of UG11 glass (Schott AG).

For each single measurement, an intensity value was put out by the respective reader, given in reflectance units (%REM). Each of the readers was able to split up the measured intensity into 1000 increments.

Generally, the higher the concentration of the anticoagulant in the sample, the less detectable substance is released from the chromogenic substrate by the corresponding blood clotting factor. Accordingly, in case of a high concentration of the anticoagulant, the absorption by the sample is low while the reflectance by the sample is high. In order to reliably evaluate the presence or absence of the anticoagulant in the sample, as a general rule, a low concentration of the anticoagulant in the sample should result in a %REM value as low as possible, while a high concentration of the anticoagulant in the sample should result in a %REM value as high as possible. In other words, the difference in reflectance units for the minimum and maximum concentration of the anticoagulant should be as large as possible.

The results for rivaroxaban and dabigatran using excitation light having a wavelength of 380 nm are listed in Tables A and B further below, and are also illustrated in Figs. 1 and 3. In order to draw a comparison with the prior art, the results for rivaroxaban and dabigatran using excitation light having a wavelength of 470 nm are illustrated in Figs. 2 and 4. The significant differences between excitation light having a wavelength of 380 nm and 470 nm are also reflected in the respective calibration curves, as shown in Figs. 5 and 6.

Table A shows the intensity values (in reflectance units, %REM) obtained from a sample (native urine) containing the direct factor Xa inhibitor rivaroxaban in concentrations of 0 ng/mL, 100 ng/mL, 200 ng/mL, 400 ng/mL, and 1500 ng/mL, measured with excitation light having a wavelength of 380 nm. In total, Table A shows the results of three measurement series taken with different readers ("Laura No. 1", "Laura No. 2", and "Laura No. 3"), wherein each measurement series is based on three single measurements for each concentration, respectively. Along with the intensity values, the calculated means and coefficients of variation (CV) are also included in Table A.

Fig. 1 illustrates the results, showing the mean for the readers "Laura No. 1", "Laura No. 2", and "Laura No. 3", as well as the mean for all readers taken together, in case of rivaroxaban. As can be taken from Fig. 1, the difference in reflectance units for the minimum and maximum concentration of the direct factor Xa inhibitor rivaroxaban (0 ng/mL vs. 1500 ng/mL) is about 400 %REM, when measured with excitation light having a wavelength of 380 nm. In contrast thereto, when measured with excitation light having a wavelength of 470 nm, the above-defined difference in reflectance units is only about 200 %REM, as can be taken from Fig. 2. Furthermore, the measurement at 470 nm showed strong fluctuations which make it almost impossible to define a cut-off value for the absence or presence of rivaroxaban (positive-negative cut-off) in the sample under investigation. Such strong fluctuations could not be observed for the measurement at 380 nm (cf. also the single intensity values for each measurement series in Table A, which are close to each other).

Table B shows the intensity values (in reflectance units, %REM) obtained from a sample (native urine) containing the direct thrombin inhibitor dabigatran in concentrations of 0 ng/mL, 100 ng/mL, 200 ng/mL, 400 ng/mL, and 1500 ng/mL, measured with excitation light having a wavelength of 380 nm. In total, Table B shows the results of three measurement series taken with different readers ("Laura No. 1", "Laura No. 2", and "Laura No. 3"), wherein each measurement series is based on three single measurements for each concentration, respectively. Along with the intensity values, the calculated means and coefficients of variation (CV) are also included in Table B.

Fig. 3 illustrates the results, showing the mean for the readers "Laura No. 1", "Laura No. 2", and "Laura No. 3", as well as the mean for all readers taken together, in case of dabigatran. As can be taken from Fig. 3, the difference in reflectance units for the minimum and maximum concentration of the direct thrombin inhibitor dabigatran (0 ng/mL vs. 1500 ng/mL) is about 400 %REM, when measured with excitation light having a wavelength of 380 nm. In contrast thereto, when measured with excitation light having a wavelength of 470 nm, the above-defined difference in reflectance units is only about 100 %REM, as can be taken from Fig. 4. Furthermore, the measurement at 470 nm showed strong fluctuations which make it almost impossible to define a cut-off value for the absence or presence of dabigatran (positive-negative cut-off) in the sample under investigation. Such strong fluctuations could not be observed for the measurement at 380 nm (cf. also the single intensity values for each measurement series in Table B, which are close to each other).

In particular, the difference in reflectance units for the minimum and maximum concentration was about 400 %REM for both rivaroxaban and dabigatran (0 ng/mL vs. 1500 ng/mL), when using excitation light having a wavelength of 380 nm. Advantageously, the intensities for rivaroxaban and dabigatran measured at a concentration of 100 ng/mL, respectively, which might be taken as the positive-negative cut-off, did not overlap, as can be taken from Tables A and B (rivaroxaban: 191 %REM as the mean of all readers vs. dabigatran: 318 %REM as the mean of all readers). Thus, using excitation light having a wavelength of 380 nm was shown to be principally suitable for the detection of both rivaroxaban and dabigatran within each sample.

In addition, excitation light having a wavelength of 380 nm could also be used for the investigation of dark urine samples. Surprisingly, despite the small overall reflectance, it was possible to observe a change of the measured intensity with the concentration of either rivaroxaban or dabigatran.

Summarizing the above results, it was demonstrated that using excitation light having a wavelength of 380 nm instead of 470 nm allowed to more clearly resolve the changes in the measured intensity of a urine sample containing rivaroxaban or dabigatran in a concentration-dependent manner. Thereby, it was possible to evaluate the presence or absence of the respective anticoagulant both with a high sensitivity and with a high specificity.

Similar results could also be obtained for the direct factor Xa inhibitors apixaban and edoxaban, showing the broad applicability of the present invention.

**Table A**

| rivaroxaban | Laura Smart No. 1 | | | Laura Smart No. 2 | | | Laura Smart No. 3 | | | all readers | |
|---|---|---|---|---|---|---|---|---|---|---|---|
| concentration (ng/mL) | single (%REM) | mean (%REM) | cv | single (%REM) | mean (%REM) | CV | single (%REM) | mean (%REM) | CV | mean (%REM) | CV |
| 0 | 159 | 145 | 8.61% | 135 | 144 | 6.69% | 140 | 142 | 4.40% | 144 | 5.97% |
| | 135 | | | 142 | | | 137 | | | | |
| | 141 | | | 154 | | | 149 | | | | |
| 100 | 192 | 181 | 10.67% | 194 | 194 | 1.30% | 202 | 199 | 1.51% | 191 | 6.59% |
| | 159 | | | 191 | | | 196 | | | | |
| | 193 | | | 196 | | | 199 | | | | |
| 200 | 254 | 276 | 19.05% | 279 | 266 | 4.90% | 304 | 315 | 7.47% | 286 | 13.01% |
| | 336 | | | 265 | | | 342 | | | | |
| | 238 | | | 253 | | | 299 | | | | |
| 400 | 346 | 387 | 9.18% | 431 | 425 | 2.45% | 423 | 387 | 13.68% | 400 | 9.38% |
| | 406 | | | 431 | | | 411 | | | | |
| | 409 | | | 413 | | | 326 | | | | |
| 1500 | 537 | 542 | 3.05% | 588 | 518 | 12.02% | 501 | 496 | 3.55% | 519 | 7.50% |
| | 528 | | | 468 | | | 510 | | | | |
| | 560 | | | 499 | | | 476 | | | | |

**Table B**

| dabigatran | Laura Smart No. 1 | | | Laura Smart No. 2 | | | Laura Smart No. 3 | | | all readers | |
|---|---|---|---|---|---|---|---|---|---|---|---|
| concentration (ng/mL) | single (%REM) | mean (%REM) | cv | single (%REM) | mean (%REM) | CV | single (%REM) | mean (%REM) | CV | mean (%REM) | CV |
| 0 | 220 | 214 | 2.60% | 220 | 211 | 7.27% | 204 | 204 | 1.23% | 210 | 4.42% |
| | 209 | | | 193 | | | 207 | | | | |
| | 213 | | | 219 | | | 202 | | | | |
| 100 | 326 | 314 | 7.56% | 372 | 345 | 6.78% | 276 | 296 | 6.93% | 318 | 9.12% |
| | 287 | | | 332 | | | 295 | | | | |
| | 330 | | | 331 | | | 317 | | | | |
| 200 | 437 | 424 | 2.82% | 363 | 375 | 3.70% | 417 | 409 | 3.18% | 402 | 6.09% |
| | 420 | | | 390 | | | 416 | | | | |
| | 414 | | | 371 | | | 394 | | | | |
| 400 | 482 | 493 | 3.75% | 464 | 486 | 6.80% | 495 | 489 | 3.05% | 489 | 4.20% |
| | 514 | | | 470 | | | 500 | | | | |
| | 482 | | | 524 | | | 472 | | | | |
| 1500 | 610 | 600 | 1.55% | 514 | 555 | 6.78% | 608 | 592 | 5.84% | 582 | 5.70% |
| | 597 | | | 563 | | | 615 | | | | |
| | 592 | | | 588 | | | 552 | | | | |

## Claims

1. Use of excitation light having a wavelength in the range of 340 nm to 400 nm for detecting at least one anticoagulant in a sample, wherein the sample is derived from a body fluid and does not contain citrated blood plasma.

2. Use according to claim 1, wherein the at least one anticoagulant is a direct factor Xa inhibitor or a direct thrombin inhibitor.

3. Use according to claim 2, wherein the direct factor Xa inhibitor is selected from the group consisting of rivaroxaban, apixaban, edoxaban, betrixaban and otamixaban, and the direct thrombin inhibitor is selected from the group consisting of dabigatran, ximelagatran, argatroban, hirudins and modified hirudins.

4. Use according to any one of claims 1 to 3, wherein the body fluid is selected from the group consisting of serum and urine.

5. A method for detecting at least one anticoagulant in a sample, comprising the steps of:
(a) providing a sample containing at least one anticoagulant, wherein the sample is derived from a body fluid and does not contain citrated blood plasma;
(b) providing a composition containing at least one blood clotting factor;
(c) providing a composition containing a chromogenic substrate conjugated to a detectable substance;
(d) mixing the sample of step (a) with the composition of step (b) and the composition of step (c) under conditions which allow the binding of the at least one anticoagulant to the at least one blood clotting factor, and which allow the at least one blood clotting factor to release the detectable substance from the chromogenic substrate; and
(e) measuring the amount of released detectable substance photometrically using excitation light having a wavelength in the range of 340 nm to 400 nm.

6. The method according to claim 5, wherein the at least one anticoagulant is a direct factor Xa inhibitor or a direct thrombin inhibitor.

7. The method according to claim 6, wherein the direct factor Xa inhibitor is selected from the group consisting of rivaroxaban, apixaban, edoxaban, betrixaban and otamixaban, and the direct thrombin inhibitor is selected from the group consisting of dabigatran, ximelagatran, argatroban, hirudins and modified hirudins.

8. The method according to any one of claims 5 to 7, wherein the body fluid is selected from the group consisting of serum and urine.

9. The method according to any one of claims 5 to 8, wherein the chromogenic substrate conjugated to a detectable substance is N-benzoyl-L-isoleucyl-L-glutamyl-L-glycyl-L-arginine-para-nitroaniline or a derivative thereof in case the at least one anticoagulant is a direct factor Xa inhibitor, or N-(para-tosyl)-glycyl-L-prolyl-L-arginine-para-nitroanilide acetate or a derivative thereof in case the at least one anticoagulant is a direct thrombin inhibitor.

10. The method according to any one of claims 5 to 9, which is a point-of-care testing.

11. The method according to claim 10, wherein the composition provided in step (b) and the composition provided in step (c) are immobilized on a test strip.

12. The method according to any one of claims 5 to 11, wherein the amount of released detectable substance is measured in step (e) using reflectance photometry.

13. The method according to any one of claims 5 to 12, wherein at least one optical filter is provided in step (e) for suppressing fluorescence light which might originate from the released detectable substance.
